(19) **Europäisches Patentamt / European Patent Office / Office européen des brevets**

(11) **EP 2 819 640 B1**

(12) **EUROPÄISCHE PATENTSCHRIFT**

(45) Veröffentlichungstag und Bekanntmachung des Hinweises auf die Patenterteilung:
**03.04.2019 Patentblatt 2019/14**

(21) Anmeldenummer: **13704567.0**

(22) Anmeldetag: **04.02.2013**

(51) Int Cl.:
*A61K 8/34* (2006.01)   *A61Q 1/02* (2006.01)
*A61Q 1/06* (2006.01)   *A61Q 1/10* (2006.01)
*A61K 8/73* (2006.01)   *A61Q 15/00* (2006.01)
*A61Q 19/00* (2006.01)   *A61K 8/02* (2006.01)
*A61Q 1/12* (2006.01)

(86) Internationale Anmeldenummer:
**PCT/EP2013/052125**

(87) Internationale Veröffentlichungsnummer:
**WO 2013/127598 (06.09.2013 Gazette 2013/36)**

(54) **VERWENDUNG VON PULVERCELLULOSE IN KOSMETIKA**

USE OF POWDERED CELLULOSE IN COSMETIC APPLICATIONS

UTILISATION DE CELLULOSE PULVÉRULENTE DANS DES COSMÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.03.2012 DE 102012203307**

(43) Veröffentlichungstag der Anmeldung:
**07.01.2015 Patentblatt 2015/02**

(73) Patentinhaber: **Evonik Degussa GmbH 45128 Essen (DE)**

(72) Erfinder:
• **WIECHERS, Susann 45239 Essen (DE)**
• **UNGER, Frank 46147 Oberhausen (DE)**
• **MEYER, Jürgen 45134 Essen (DE)**

(74) Vertreter: **Evonik Patent Association c/o Evonik Industries AG IP Management Bau 1042A/PB 15 Paul-Baumann-Straße 1 45764 Marl (DE)**

(56) Entgegenhaltungen:
EP-A1- 1 057 477     EP-A1- 2 389 920
WO-A1-01/89466      WO-A1-97/32560
US-A- 3 278 383      US-A- 3 530 217
US-A- 4 857 352      US-A1- 2005 002 996
US-A1- 2007 062 009

• SHCHERBAKOVA T P ET AL: "Comparative study of powdered and microcrystalline cellulose samples of a various natural origins: Physical and chemical characteristics", RUSSIAN JOURNAL OF BIOORGANIC CHEMISTRY, SP MAIK NAUKA/INTERPERIODICA, DORDRECHT, Bd. 38, Nr. 7, 30. November 2012 (2012-11-30), Seiten 689-696, XP035147539, ISSN: 1608-330X, DOI: 10.1134/S1068162012070187
• Florence H. Forziati, Walter K. Stone, John W. Rowen, and William D. Appel: "Cotton Powder for Infrared Transmission Measurements", Journal of Research of the National Bureau of Standards, Bd. 45, Nr. 2 1. August 1950 (1950-08-01), Seiten 109-113, XP055126291, Gefunden im Internet: URL:http://nvlpubs.nist.gov/nistpubs/jres/045/2/V45.N02.A01.pdf [gefunden am 2014-07-01]
• TAKUYA ISOGAI ET AL: "Degrees of polymerization (DP) and DP distribution of cellouronic acids prepared from alkali-treated celluloses and ball-milled native celluloses by TEMPO-mediated oxidation", CELLULOSE, KLUWER ACADEMIC PUBLISHERS (DORDRECHT), NL, Bd. 16, Nr. 1, 6. August 2008 (2008-08-06), Seiten 117-127, XP019640464, ISSN: 1572-882X

EP 2 819 640 B1

- Arne Lützen, Burkhard Fugmann: "Cellulose", Römpp Lexikon , December 2009 (2009-12), Retrieved from the Internet: URL:https://roempp.thieme.de/roempp4.0/do/data/RD-03-00833 [retrieved on 2017-03-24]
- Wolfgang Türk: "Linters", Römpp Lexikon , May 2005 (2005-05), Retrieved from the Internet: URL:https://roempp.thieme.de/roempp4.0/do/data/RD-12-01243 [retrieved on 2017-03-24]

- Wolfgang Türk, Römpp Redaktion: "Baumwolle", Römpp Lexikon , December 2007 (2007-12), Retrieved from the Internet: URL:https://roempp.thieme.de/roempp4.0/do/data/RD-02-00425 [retrieved on 2017-03-24]

- Arne Lützen, Burkhard Fugmann: "Cellulose", Römpp Lexikon , December 2009 (2009-12), Retrieved from the Internet: URL:https://roempp.thieme.de/roempp4.0/do/

- Wolfgang Türk, Römpp Redaktion: "Baumwolle", Römpp Lexikon , December 2007 (2007-12), Retrieved from the Internet: URL:https://roempp.thieme.de/roempp4.0/do/data/RD-02-00425 [retrieved on 2017-03-24]

**Beschreibung**

Gebiet der Erfindung

Gegenstand der Erfindung ist eine kosmetische Formulierung wie in Anspruch 1 beschrieben enthaltend feste Partikel mit einer mittleren Partikelgröße von 3 $\mu$m bis 15 $\mu$m, dadurch gekennzeichnet, dass die Partikel mindestens 95 Gew.-% native, aus Pflanzenfasern gewonnene Cellulose enthalten, wobei sich die Gewichtsprozente auf das trockene Partikelgesamtgewicht beziehen.

Stand der Technik

Es ist bekannt, dass mikrokristalline Cellulosen und Cellulosederivate in der Kosmetik eingesetzt werden, um kosmetische Formulierungen zu stabilisieren.
EP1036799 beschreibt die Herstellung von Gel-Dispersionen von unmodifizierter und modifizierter Cellulose mit einer Fraktion an Cellulose-I-Typ-Kristallkomponente von nicht mehr als 0,1 und einer Fraktion an Cellulose-II-Typ-Kristallkomponente von nicht mehr als 0,4. Die schwefelsaure wässrige Lösung der Cellulose wird mit Wasser verdünnt, so dass eine gelartige Cellulose-Suspension mit maximal 6 % Cellulose entsteht. Trockene Cellulosepartikel oder Cellulosekompositpartikel können durch Sprühtrocknung der zuvor in wässriger Schwefelsäure gelösten Cellulose erhalten werden.
EP0264853 beschreibt die Herstellung von kovalent vernetzten Cellulosepartikeln, die nicht größer als 300 $\mu$m sind. Die Partikel werden modifiziert, z.B. zu Cellulose Xanthanat, um die Vernetzung durchführen zu können.
EP1545433 offenbart die Herstellung von kosmetisch verwendbaren Partikeln, deren Kern eine fluoreszierende Komponente enthält und zusätzlich ein Coating aus vernetztem Polyvinylalkohol besitzt. Als solchermaßen modifiziertes Kernmaterial wird unter anderem Cellulose aufgeführt. Die Partikel sollen durch Absorption von UV-Licht und Emittierung von sichtbarem Licht Hautunebenheiten optisch ausgleichen.
In WO2009085444 werden unter anderem sphärische Cellulose-Kugeln als Makropartikel genutzt, um zusammen mit Nanopartikeln ein fraktales Netzwerk zu bilden. Dies führt zur Ausbildung von Gelstrukturen in kosmetischen Formulierungen, die dazu genutzt werden, Hautunebenheiten durch den "Soft focus effect" optisch zu reduzieren.
EP1299069 beschreibt die Herstellung von stabilen Multiphasen-Emulsionen, in denen sich Partikel in der diskontinuierlichen Phase befinden. Modifizierte Cellulosen werden als Cellulosepulver beschrieben und als Varianten unter vielen verschiedenen Füllstoffen genannt.
EP1372576 beschreibt den Einsatz modifizierter Cellulosen und Cellulosederivate in selbstschäumenden oder schaumförmigen kosmetischen Formulierungen.
In WO2009112375 werden Emulsionen beschrieben, bei denen die Phasengrenzen durch in der Phasengrenzfläche befindlichen modifizierten biopolymeren Mikropartikel stabilisiert werden. Als geeignete Mikropartikel werden ausschließlich kovalent modifizierte Cellulosen beschrieben.
Aufgabe der Erfindung war es, einen natürlichen Inhaltsstoff für kosmetische Formulierungen zur Verfügung zu stellen, der die Klebrigkeit von Formulierungen, insbesondere enthaltend feuchtigkeitsspendende Polyole wie z.B. Glycerin, zu reduzieren vermag.

Beschreibung der Erfindung

Überraschenderweise wurde gefunden, dass native, nicht derivatisierte, nicht kovalent modifizierte Cellulosepartikel die gestellte Aufgabe lösen.
Sie erzeugen ein angenehmes Hautgefühl.
Gegenstand der vorliegenden Erfindung sind daher kosmetische Formulierungen enthaltend feste Partikel wie in Anspruch 1 beschrieben.
Ein weiterer Gegenstand der Erfindung ist die Verwendung von solch festen Partikeln zur Herstellung einer kosmetischen Formulierung.
Ein Vorteil der vorliegenden Erfindung ist, dass sie ein angenehmes, nicht klebriges Hautgefühl erzeugen.
Noch ein Vorteil der vorliegenden Erfindung ist, dass die Formulierungen homogene und harmonische Texturen zu erzeugen vermögen. So bewirken die erfindungsgemäßen Cellulosepartikel eine bessere Verteilbarkeit kosmetischer Formulierungen auf der Haut und ein verbessertes Einzugsvermögen bei gleichzeitig reduzierter Glitschigkeit und Klebrigkeit.
Noch ein Vorteil der vorliegenden Erfindung ist, dass die Partikel eine Stabilisierung von mehrphasigen Systemen, wie beispielsweise einer kosmetischen Emulsion, bewirken können.
Durch die Darreichungsform der Partikel als trockene Partikel mit einem hohem Celluloseingehalt wird der Eintrag unerwünschter Nebenprodukte wie z.B. bei Cellulosedispersionen oder Kompositmaterialien verhindert. Nebenprodukte sind besonders bei der Verwendung von Inhaltsstoffen in der Kosmetik aus gesundheitlichen Gründen unerwünscht, zudem können sie die Stabilität der Formulierung negativ beeinträchtigen.

Weiterhin ist es von Vorteil, dass die erfindungsgemäßen nativen Cellulosepartikel nicht durch chemische Modifikationen verändert wurden und ihnen auch keine chemisch modifizierten Cellulosebestandteile beigemischt wurden. Dadurch vermögen die erfindungsgemäßen Partikel den Wunsch vieler Konsumenten nach möglichst natürlichen kosmetischen Inhaltsstoffen sehr gut zu entsprechen.

Noch ein Vorteil der vorliegenden Erfindung ist, dass die Partikel nicht oder nur sehr gering den Viskostitätsaufbau einer kosmetischen Formulierung beeinflussen.

Noch ein Vorteil der vorliegenden Erfindung ist, dass die Partikel hohe Mengen Öl zu binden vermögen.

Ein weiterer Vorteil der vorliegenden Erfindung ist, dass die Partikel eine homogene Verteilbarkeit und angenehmeres Applikationsverhalten, insbesondere bei Formulierungen auf Basis polyacrylatbasierter Verdicker, bewirken.

Ein Gegenstand der vorliegenden Erfindung ist eine kosmetische Formulierung, die eine Emulsion ist und die Glycerin und feste Partikel mit einer mittleren Partikelgröße von 3 μm bis 15 μm, besonders bevorzugt von 4 μm bis 10 μm, enthält, die dadurch gekennzeichnet ist, dass die Partikel zu mindestens 95 Gew.-%, bevorzugt mindestens 97 Gew.-%, besonders bevorzugt mindestens 99 Gew.-% native, aus Pflanzenfasern gewonnene Cellulose, enthalten, wobei sich die Gewichtsprozent auf das trockene Partikelgesamtgewicht beziehen.

Höhere Reinheitsgrade als 99,99 Gew.-% native, aus Pflanzenfasern gewonnene Cellulose bezogen auf das Gesamtpartikelgewicht sind wirtschaftlich nicht vertretbar.

Unter dem Begriff "kosmetische Formulierung" ist im Zusammenhang mit der vorliegenden Erfindung eine Zusammensetzung zu verstehen, die neben den festen Partikeln mindestens eine Komponente enthält, ausgewählt aus Wasser, kosmetischen Ölen und kosmetischen Wachsen. Bevorzugt enthalten die erfindungsgemäßen Formulierungen mindestens einer weitere Komponente ausgewählt aus Emollients, Emulgatoren, Verdickern/Viskositätsreglern/Stabilisatoren, Polyolen und kosmetischen Wirkstoffen. Vertreter der einzelnen Gruppen sind dem Fachmann bekannt und können beispielsweise der deutschen Anmeldung DE 102008001788.4 entnommen werden. Besonders bevorzugt sind die erfindungsgemäßen Formulierungen wässrige Formulierungen, wobei unter dem Bergriff "wässrig" bezogen auf die Gesamtformulierung ein Wassergehalt von 5 Gew.-% bis 95 Gew.-%, bevorzugt 10 Gew.-% bis 90 Gew.-%, besonders bevorzugt 20 Gew.-% bis 80 Gew.-%, verstanden wird.

Unter dem Begriff "native, aus Pflanzenfasern gewonnene Cellulose" ist im Zusammenhang mit der vorliegenden Erfindung eine Cellulose zu verstehen, die keine chemische Modifikation in Form einer Säure- oder Basebehandlung erfahren hat, durch die die amorphen Anteile der Cellulose mindestens teilweise entfernt werden, und insbesondere keine chemische Derivatisierung wie z.B. Hydroxypropylierung, Hydroxyethylierung, Carboxymethylierung, Veresterung (z.B. Acetylierung), Veretherung (z.B. Methylierung) und Quaternierung erfahren hat, sondern lediglich aus einem Naturstoff über Vermahlen im Wässrigen erhalten wurde.

Unter dem Begriff "trocken" ist im Zusammenhang mit der vorliegenden Erfindung eine Cellulose beschrieben, die folgende Trocknung durchlaufen hat:

10 g der Cellulose werden bei 105 °C 2 Stunden im Trockenschrank gelagert.

Die Restfeuchte beträgt vor der Trocknung maximal 9 %.

Nach Abkühlung auf Raumtemperatur im Exsikkator wird die Probe ausgewogen. Berechnung:

$$100\ \% - ((\text{Gewicht nach Trocknung (g)} * 100\ \%)/\text{Einwaage vor Trocknung (g)}) = (\%)\ \text{Restfeuchte}$$

Unter dem Begriff "fest" ist im Zusammenhang mit der vorliegenden Erfindung der Aggregatzustand "fest" bei einer Umgebungstemperatur, bei der kosmetische Formulierungen eingesetzt werden, zu verstehen, diese Temperaturbereich erstreckt sich insbesondere von 15 °C bis 45 °C.

Alle Bedingungen wie beispielsweise Druck und Temperatur sind, wenn nicht anders angegeben, Standardbedingungen (25 °C, 1 bar). Prozentzahlen sind, soweit nicht anders beschrieben, in Massenprozent angegeben.

Um die mittlere Partikelgröße zu bestimmen, wird die Laserbeugung eingesetzt. Dazu wird die zu untersuchende Probe in deionisiertem Wasser mit Hilfe eines Ultraschallbades (Bandelin SONOREX TK 30; Dispergierdauer 5 Minuten) dispergiert. Die Messung wird mit dem Lasergranulometer LS 230 von Beckman-Coulter vorgenommen. Das Material wird mit dem Nassmodul SVM vermessen, die Berechnung der Partikelparameter erfolgt nach der Fraunhofer-Theorie. Die mittlere Partikelgröße stellt den d50-Wert bei einer volumengewichteten Partikelgrößenverteilung dar.

Der Gehalt an Cellulose in den Cellulosepartikeln wird folgendermaßen bestimmt:

2,5 g zerkleinerte Probe (Trockengehalt in einer gesonderten Probe ermitteln: 100% - TV % (TV: Trocknungsverlust)) werden in ein 150 ml Becherglas eingewogen (E). Mittels Pipette werden 30 ml auf 20 °C temperierte 17,5%-ige Natronlauge zur Probe gegeben. Die Masse wird mit einem Glasstab, dessen eines Ende abgeplattet ist, vorsichtig zerdrückt und 30 Minuten stehengelassen. Nach dieser Zeit wird mit 100 ml RO (*reverse* osmosis)-Wasser schnell verdünnt, sofort umgerührt und abgesaugt. Die zu verwendende Glasfritte G3 ist vorher im Trockenschrank zu trocknen, im Exsikkator abzukühlen und zu wiegen ($B_{leer}$). Es ist streng darauf zu achten, dass die verdünnte Masse so rasch wie möglich von der alkalischen Flüssigkeit abgesaugt und auch das anschließende Waschen zügig durchgeführt wird. Das Auswaschen erfolgt mit RO-Wasser portionsweise. Es wird solange gewaschen, bis gegenüber pH-Papier keine alkalische Reaktion mehr eintritt. Hierauf wird der Filterkuchen mit 0,5%-iger Salzsäure übergossen und 10 Minuten ohne Absaugen stehengelassen. Dann wird wieder mit RO-Wasser gewaschen, bis das pH-Papier keine Säurereaktion mehr zeigt. Die Durchführung findet bei 20 °C statt. Danach wird die Fritte bei 105 °C bis zur Gewichtskonstanz getrocknet, im Exsikkator abgekühlt und ausgewogen ($B_{Ausw}$).

$$\text{Gehalt Cellulose (\%)} = \frac{(B_{Ausw} - B_{leer}) \times 100 \times 100}{E \times (100-TV)}$$

Grundlegender Unterschied zu den herkömmlich in Kosmetika als Verdicker eingesetzten mikrokristallinen Cellulosen zu der hier eingesetzten Cellulose ist die Eigenschaft, dass die Partikel schwer wasserlöslich sind und als feste Partikel in der kosmetischen Formulierung wirken. Daher sind erfindungsgemäß insbesondere kosmetische Formulierungen bevorzugt, die dadurch gekennzeichnet sind, dass die Partikel eine maximale Löslichkeit in Wasser bei pH 7,0, 20 °C, 1 bar, von 0 g/L bis 0,5 g/L, bevorzugt von 0 g/L bis 0,2 g/L, besonders bevorzugt von 0 g/L bis 0,08 g/L aufweisen.

Die in den erfindungsgemäßen Formulierungen eingesetzten Partikel weisen bevorzugt eine Cellulose eines Kristallinitätsgrades von 40 bis 90 %, bevorzugt von 50 bis 85%, besonders bevorzugt von 60 bis 80 %auf.
Für die quantitative Bestimmung der Kristallinität von Zellulose-Proben wird folgende "peak height"-Methode verwendet, beschrieben zum Beispiel bei. N. Terinte, R. Ibbett und K. C. Schuster, Lenzinger Berichte 89 (2011) 118-131:
Es werden Röntgenbeugungsaufnahmen im Bereich von 5°-45° (2 $\Theta$) in Reflexion erstellt.
Die Luftstreuungskurve wird mittels des rein-kristallinen Standards NIST640c bestimmt, und diese als Untergrund für die Röntgenbeugungs-Diagramme der Messproben benutzt. Dieser Untergrund wird von der Messprobe abgezogen. Der Kristallinitätsgrad CI wird als Verhältnis der Peakhöhe von dem kristallinen Signal I(002) bei 22° (2 $\Theta$) nach dem Abziehen des nicht-kristallinen Beitrags I(nicht-kristallin) (das Signal bei 18° (2 $\Theta$) und der Peakhöhe des kristallinen Peaks I(002) bei 22° (2 $\Theta$) berechnet:

$$CI = ((I(002) - I(\text{nicht-kristallin}))/I(002)) \times 100 \text{ \%}$$

Erfindungsgemäß werden kosmetische Formulierungen bevorzugt, die dadurch gekennzeichnet sind, dass der kristalline Anteil der in den Partikeln enthaltenen Cellulose zum Großteil nicht dem Cellulose Typ II entspricht. Insbesondere ist der Gehalt von Cellulose Typ I im kristallinen Anteil bevorzugt größer als 95 Gew.-%, besonders bevorzugt größer als 99 Gew.-% bezogen auf die Gesamtkristallinität. Die verschiedenen Cellulosetypen sind zum Beispiel beschrieben bei Park et al. Biotechnology for Biofuels 2010, 3:10. Die Bestimmung des Cellulose-Typs wurde aufgrund eines Abgleichs der Röntgenbeugungsdiagramme mit den in der ICSD-Datenbank (Inorganic Crystal Structure Database) vorhandenen Referenzdiagrammen durchgeführt. Dieser Abgleich erfolgte aufgrund der Peaklagen und Intensitätsverhältnissen mit Hilfe der Softwaresuchfunktion von HighScore Plus (Hersteller: PANalytical) in der Version: 3.0c.
Erfindungsgemäß werden kosmetische Formulierungen bevorzugt, die dadurch gekennzeichnet sind, dass die in den Partikeln enthaltene Cellulose einen mittleren Polymerisierungsgrad von 1 bis 50000, bevorzugt von 50 bis 20000, besonders bevorzugt von 200 bis 3000.
Der mittlere Polymerisierungsgrad wird über die Messung der relativen Viskosität der in einer Cuen (Kupfer(II)ethylendiamin)-Lösung gelösten Cellulose wie folgt ermittelt. Einwaage von 1,3 g Probe in einen 100 ml Erlenmeyerkolben ein (WS). Der Trockengehalt der Probe (TV) bzw. die Restfeuchte ist separat zu bestimmen. Nachspülen mit 25 ml RO-Wasser, das vor der Verwendung mit Stickstoff gespült ist, und Verteilen der Cellulose im Wasser durch Schwenken.
Zufügen von 25 ml 1M Cuen-Lösung, die vor der Verwendung mit Stickstoff gespült ist. Einleiten von Stickstoff in die Probelösung. Verschließen des Erlenmeyerkolben mit dem dazugehörigen Glasstopfen. Schütteln der Probe, bis sich die Cellulose vollständig aufgelöst hat. Überführung von ca. 15 ml dieser Lösung in ein Ubbelohde Viskosimeter mit der Kapillare 1c. Temperieren der Probelösung auf 25°C $\pm$ 0,1°C. Mit einer Pipetteierhilfe wird die Lösung durch die Viskosimeterkapillare bis über die obere Glaskugel gezogen. Stoppen der Zeit, die die Lösung benötigt, um von der oberen zur unteren Markierung zu fließen. Wiederholung des Vorgangs und Berechnung aus beiden Zeitnahmen des Mittelwerts t1, falls beide Werte nicht mehr als 1% voneinander abweichen. Ansonsten ist eine weitere Bestimmung durchzuführen, der Mittelwert zweier nicht mehr als 1% voneinander abweichenden Ergebnisse ist zu bestimmen. Wiederholung des Vorgangs ohne Cellulose zur Bestimmung des Blindwerts. Hierzu wird die Kapillare 1 verwendet. Der erhaltene Mittelwert t2 ist die Fließzeit der reinen Cuen-Lösung.
Berechnung der relativen Viskosität:

$$\eta rel=(t1*k1)/(t2*k2)$$

$t_1 =$ Fließzeit der Probelösung (Mittelwert)

$t_2 =$ Fließzeit der Cuen-Lösung (Mittelwert)

$k_1 =$ Konstante des Ubbelohde Viskosimeters, Kapillare 1c

$k_2 =$ Konstante des Ubbelohde Viskosimeters, Kapillare 1

Aus der Tabelle 0315.-1. in Ph. Eur. 6.3. Powdered Cellulose wird der Wert für die intrinsische Viskosität zur relativen Viskosität für die Lösung der Cellulose entnommen. Der Polymerisierungsgrad wird als

$$PG=(9500*\eta_C)/E*(100-TV)$$

berechnet, wobei
$\eta_c$: intrinsische Viskosität, E: Einwaage, TV: Trocknungsverlust in % ist.
Erfindungsgemäß werden kosmetische Formulierungen bevorzugt, die dadurch gekennzeichnet sind, dass die enthaltenen festen Partikel eine Schüttdichte von 100-300 g/L, bevorzugt 120-270 g/L, besonders bevorzugt 140-240 g/L besitzen. Die Schüttdichte wird bestimmt in nach DIN 53468.
Es ist erfindungsgemäß bevorzugt, wenn die kosmetische Formulierung bezogen auf die Gesamtformulierung 0,01 Gew.-% bis 30 Gew.-%, bevorzugt 0,05 Gew.-% bis 20 Gew.-%, besonders bevorzugt 0,1 Gew.-% bis 10 Gew.-% Partikel enthält.
Da sich die Partikel vorteilhaft in Mehrphasensystemen einsetzten lassen, sind erfindungsgemäß bevorzugte Formulierungen fließfähige Formulierungen, insbesondere O/W- oder W/O-Emulsionen, mit einer Viskosität von 0,01 Pas bis 100000 Pas, bevorzugt von 1 Pas bis 20000 Pas, besonders bevorzugt von 10 Pas, insbesondere 25 Pas, bis 10000 Pas ist, wobei diese Viskosität bei einer Scherrate von 10 s$^{-1}$ und bei 20 °C gemessen wird.
Besonders gute Ergebnisse sind in kosmetischen Formulierungen erzielt worden, wenn die Partikel ein Ölabsorptionsvermögen von 1,0 g bis 2,5 g, bevorzugt von 1,2 g bis 2,2 g, besonders bevorzugt von 1,5 g bis 2,0 g Cyclopentasiloxane pro g trockenem Partikel aufweisen.
In einer alternative Ausführungsform der vorliegenden Erfindung sind ebenso besonders gute Ergebnisse in kosmetischen Formulierungen erzielt worden, wenn die Partikel ein Ölabsorptionsvermögen von 1,0 g bis 2,0 g, bevorzugt von 1,2 g bis 1,8 g, besonders bevorzugt von 1,5 g bis 1,7 g Diethyhexylcarbonate pro g trockenem Partikel aufweisen.
In einer alternative Ausführungsform der vorliegenden Erfindung sind ebenso besonders gute Ergebnisse in kosmetischen Formulierungen erzielt worden, wenn, die Partikel ein Ölabsorptionsvermögen von 1,0 g bis 2,0 g, bevorzugt von 1,1 g bis 1,7 g, besonders bevorzugt von 1,4 g bis 1,6 g Isopropyl-Myristate pro g trockenem Partikel aufweisen.
In einer alternative Ausführungsform der vorliegenden Erfindung sind ebenso besonders gute Ergebnisse in kosmetischen Formulierungen erzielt worden, wenn, die Partikel ein Ölabsorptionsvermögen von 1,0 g bis 2,0 g, bevorzugt von 1,2 g bis 1,8 g, besonders bevorzugt von 1,45 g bis 1,7 g Caprylic/Capric Triglyceride pro g trockenem Partikel aufweisen.
In einer alternative Ausführungsform der vorliegenden Erfindung sind ebenso besonders gute Ergebnisse in kosmetischen Formulierungen erzielt worden, wenn, die Partikel ein Ölabsorptionsvermögen von 1,0 g bis 2,5 g, bevorzugt von 1,2 g bis 2,2 g, besonders bevorzugt von 1,5 g bis 2,0 g Mineralöl pro g trockenem Partikel aufweisen.
Erfindungsgemäß besonders bevorzugte kosmetische Formulierungen weisen Partikel mit einem Ölabsorptionsvermögen für Cyclopentasiloxane, Diethyhexylcarbonate, Isopropyl Myristate, Caprylic/Capric Triglyceride und Mineralöl mit oben genannten besonders bevorzugten Bereichen auf.
Es hat sich ebenso vorteilhaft erwiesen, wenn die erfindungsgemäßen kosmetischen Formulierungen Partikel enthalten, welche ein Wasserabsorptionsvermögen von 1 g bis 3 g, bevorzugt von 1,5 g bis 3,5 g, besonders bevorzugt von 1,7 g bis 2,3 g Wasser von pH 7 pro g trockenem Partikel aufweisen.
Ganz besonders bevorzugt sind die erfindungsgemäßen kosmetischen Formulierungen, welche Partikel mit einem Ölabsorptionsvermögen für Cyclopentasiloxane, Diethyhexylcarbonate, Isopropyl Myristate, Caprylic/Capric Triglyceride und Mineralöl mit oben genannten besonders bevorzugten Bereichen und einem Wasserabsorptionsvermögen von 1,7 g bis 2,3 g Wasser von pH 7 pro g trockenem Partikel enthalten.
Bestimmt wird das Öl-/Wasserabsorptionsvermögen nach dem folgenden Verfahren: Es werden 2 g Partikel in eine Petrischale abgewogen. Es wird die jeweilige Flüssigkeit (Öl oder Wasser) in eine Tropfflasche abgefüllt und gewogen. Es erfolgt eine tropfenweise Zugabe der Flüssigkeit und Mischen der Partikel und der Flüssigkeit mit einem Spatel, bis ein feuchter Glanz auf den Partikeln zu sehen ist und keine weiter Flüssigkeit mehr absorbiert werden kann. Die benötigte Ölmenge wird durch Rückwiegen der Tropfflasche ermittelt.

Die erfindungsgemäßen Formulierungen enthalten Glycerin, da die Partikel dem negativen Hautgefühl dieser Komponente vorteilhaft entgegenwirken können. Ganz besonders bevorzugt enthält die erfindungsgemäße Formulierung Glycerin in einer Menge von 0,001 Gew.-% bis 20 Gew.-%, bevorzugt 0,01 Gew.-% bis 15 Gew.-%, besonders bevorzugt 0,1 Gew.-% bis 10 Gew.-% bezogen auf die Gesamtformulierung.

Die Verwendung von den im Zusammenhang mit den erfindungsgemäßen Formulierungen genannten Partikeln zur Herstellung von Kosmetika leistet einen Beitrag zur Lösung der oben genannten Aufgabe. Somit ist ebenfalls ein Gegenstand der vorliegenden Erfindung die Verwendung von festen Partikel mit einer mittleren Partikelgröße von 3 μm bis 15 μm, besonders bevorzugt von 4 μm bis 10 μm, zur Herstellung einer erfindungsgemäßen kosmetischen Formulierung, dadurch gekennzeichnet, dass die Partikel zu mindestens 95 Gew.-%, bevorzugt mindestens 97 Gew.-%, besonders bevorzugt mindestens 99 Gew.-% aus nativer, aus Pflanzenfasern gewonnener Cellulose bestehen, wobei sich die Gewichtsprozent auf das trockene Partikelgesamtgewicht beziehen.

Bei dieser erfindungsgemäßen Verwendung werden insbesondere bevorzugte feste Partikel verwendet, die in den erfindungsgemäßen Formulierungen als bevorzugt enthaltene oben vorbeschrieben sind.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll..

Das Hautgefühl der in den folgenden Beispielen beschriebenen kosmetischen Formulierungen wurde durch ein sogenanntes Panel bestimmt. Mindestens fünf Personen verglichen die sensorischen Eigenschaften der kosmetischen Formulierungen und der jeweiligen Vergleichsformulierung ohne die Zusammensetzung zu kennen. Es werden die Eigenschaften aufgeführt, die die Mehrheit der Personen bevorzugt beschrieben hat.

Der zur Auswertung der Emulsionsvergleichsbeispiele verwendeten Nomenklatur zum Thema "Stabilität" liegen folgende Anforderungen zugrunde. Ist die Stabilität mit "gut" bewertet, so bedeutet das, dass eine solche Emulsion mindestens einen Monat bei Raumtemperatur, -5°C und 40°C stabil ist. "Stabil" bedeutet dabei, dass keinerlei Öl- oder Wasserabscheidung auftritt, dass das Erscheinungsbild der Emulsion homogen bleibt und dass in der Emulsion keine nennenswerten Veränderungen von Viskosität, Farbe oder Geruch auftreten.

*Beispiel 1 und Vergleichsbeispiel V1: Öl-in-WasserSonnenschutzspray mit hohem Sonnenschutzfaktor.*

Es wurden die in Tabelle 1 angegebenen Formulierungen hergestellt und deren Stabilität, Erscheinungsbild und Hautgefühl bewertet.

Tabelle 1 gemäß Beispiel 1: Formulierungen und Ergebnisse von Beispiel 1 und Vergleichsbeispiel V1, ÖI-in-Wasser Sonnenschutzspray mit hohem Sonnenschutzfaktor.

| Beispiel | 1 | V1 |
|---|---|---|
| TEGO® Alkanol S 20 P (Evonik Industries AG) (Steareth-20) | 1,00 % | 1,00 % |
| TEGOSOFT® TN (Evonik Industries AG) (C12-15 Alkyl Benzoate) | 6,00 % | 6,00 % |
| TEGOSOFT® TIS (Evonik Industries AG) (Triisostearin) | 1,50 % | 1,50 % |
| REWOPAL® PIB 1000 (Evonik Industries AG) (Polyisobutene) | 1,00 % | 1,00 % |
| TEGO® Sun T 805 (Evonik Industries AG) (Titanium Dioxide; Trimethoxycaprylylsilane) | 1,50 % | 1,50 % |

(fortgesetzt)

| Beispiel | 1 | V1 |
|---|---|---|
| Bis-Ethylhexyloxyphenol Methoxyphenyl Triazin | 5,00 % | 5,00 % |
| Butyl Methoxydibenzoylmethane | 5,00 % | 5,00 % |
| Diethylhexyl Butamido Triazone | 2,00 % | 2,00 % |
| Ethylhexyl Methoxycinnamate | 1,00 % | 1,00 % |
| Ethylhexyl Salicylate | 5,00 % | 5,00 % |
| Homosalate | 6,00 % | 6,00 % |
| Octocrylene | 5,00 % | 5,00 % |
| TEGO® Carbomer 341 ER (Evonik Industries AG) (Acrylates/C10-30 Alkyl Acrylate Crosspolymer) | 0,50 % | 0,50 % |
| Tocopheryl Acetate | 0,50 % | 0,50 % |
| Ultrafeine Cellulose Arbocel M8, J. Rettenmaier und Söhne GmbH + Co. KG | 1,00 % | 0,00 % |
| Glycerin | 5,00 % | 5,00 % |
| Wasser | ad 100 % | ad 100 % |
| Phenylbenzimidazol Sulfonic Acid (20 % in Wasser) | 10,00 % | 10,00 % |
| EDTA | 0,10 % | 0,10 % |
| Ethanol | 5,00 % | 5,00 % |
| Tris(hydroxymethyl)-aminomethan (30 % in Wasser) | 1,50 % | 1,50 % |
| Phenoxyethanol; Ethylhexylglycerin | q.a. | q.a. |
| Stabilität | Gut | Gut |
| Erscheinungsbild | Gelblich, homogen | Gelblich, homogen |
| Hautgefühl | Deutlich reduzierte Klebrigkeit, geringere Öligkeit, gute Absorption. | Sehr klebrig, ölig, schlechte Absorption. |

*Beispiel 2 und Vergleichsbeispiel V2: Öl-in-Wasser After Shave Cream*

Es wurden die in Tabelle 2 angegebenen Formulierungen hergestellt und deren Stabilität, Erscheinungsbild und Hautgefühl bewertet.

Tabelle 2 gemäß Beispiel 2: Formulierungen und Ergebnisse von Beispiel 2 und Vergleichsbeispiel V2, ÖI-in-Wasser After Shave Cream.

| Beispiel | 2 | V2 |
|---|---|---|
| Axol® C 62 (Evonik Industries AG) (Glyceryl Stearate Citrate) | 1,50 % | 1,50 % |
| TEGIN® M (Evonik Industries AG) (Glyceryl Stearate) | 2,00 % | 2,00 % |
| TEGO® Alkanol 1618 (Evonik Industries AG) (Cetearyl Alkohol) | 3,00 % | 3,00 % |
| TEGOSOFT® CT (Evonik Industries AG) (Caprylic/Capric Triglyceride) | 3,50 % | 3,50 % |
| TEGOSOFT® CR (Evonik Industries AG) (Cetyl Ricinoleate) | 2,00 % | 2,00 % |

(fortgesetzt)

| Beispiel | 2 | V2 |
|---|---|---|
| TEGOSOFT® TIS (Evonik Industries AG) (Triisostearin) | 1,00 % | 1,00 % |
| TEGOSOFT® MM (Evonik Industries AG) (Myristyl Myristate) | 0,50 % | 0,50 % |
| Cyclopentasiloxane | 4,00 % | 4,00 % |
| Jojobaöl | 2,00 % | 2,00 % |
| Tocopheryl Acetate | 0,50 % | 0,50 % |
| Cellulosepartikel aus Beispiel 1 | 2,00 % | 0,00 % |
| | | |
| Glycerin | 4,00 % | 4,00 % |
| Panthenol | 0,50 % | 0,50 % |
| Allantoin | 0,20 % | 0,20 % |
| Wasser | ad 100 % | ad 100 % |
| | | |
| TEGO® Carbomer 134 (Evonik Industries AG) (Carbomer) | 0,30 % | 0,30 % |
| Tegosoft® OS (Evonik Industries AG) (Ethylhexyl Stearate) | 1,20 % | 1,20 % |
| Bisabolol | 0,50 % | 0,50 % |
| 10%ige wässrige Natronlauge | 0,90 % | 0,90 % |
| Methylisothiazolinone, Methylparaben Ethylparaben | q.a. | q.a. |
| | | |
| Stabilität | Gut | Gut |
| Erscheinungsbild | Weiß, homogen | Weiß, homogen |
| Hautgefühl | Leicht verteilbar, schnelle Absorption, wenig Rückstand. | Schwer verteilbar, langsame Absorption, fettiger Rückstand |

*Beispiel 3 und Vergleichsbeispiel V3: Natürliche Öl-in-Wasser Natürliche Creme*

Es wurden die in Tabelle 3 angegebenen Formulierungen hergestellt und deren Stabilität, Erscheinungsbild und Hautgefühl bewertet.

Tabelle 3 gemäß Beispiel 3: Formulierungen und Ergebnisse von Beispiel 3 und Vergleichsbeispiel V3, Natürliche Öl-in-Wasser Creme.

| Beispiel | 3 | V3 |
|---|---|---|
| TEGO® Care PSC3 (Evonik Industries AG) (Polyglyceryl-3 Dicitrate/Stearate) | 2,50 % | 2,50 % |
| TEGIN® M (Evonik Industries AG) (Glyceryl Stearate) | 1,20 % | 1,20 % |
| TEGO® Alkanol 18 (Evonik Industries AG) (Stearyl Alkohol) | 1,30 % | 1,30 % |

(fortgesetzt)

| Beispiel | 3 | V3 |
|---|---|---|
| TEGOSOFT® P (Evonik Industries AG) (Isopropyl Palmitate) | 6,50 % | 6,50 % |
| TEGOSOFT® TIS (Evonik Industries AG) (Triisostearin) | 3,50 % | 3,50 % |
| Mandelöl (Prunus Dulcis) | 6,00 % | 6,00 % |
| Cellulosepartikel aus Beispiel 1 | 1,00 % | 0,00 % |
| Glycerin | 10,00% | 10,00 % |
| Wasser | ad 100,00 % | ad 100 % |
| 10%ige wässrige Natronlauge | 0,20 % | 0,20 % |
| Rokonsal BSB-N (ISP) (Benzyl Alkohol, Glycerin, Benzoic Acis, Sorbic Acid) | 0,80 % | 0,80 % |
| Stabilität | Gut | Gut |
| Erscheinungsbild | Weiß, homogen | Weiß, homogen |
| Hautgefühl | Reduzierte Klebrigkeit, geringere Öligkeit, gute Absorption, wenig Rückstand. | Klebrig, öliger/feuchter Rückstand, schlechte Absorption. |

*Beispiel 4 und Vergleichsbeispiele V4.1, V4.2 und V4.3 und V 4.4 Emulgatorfreie Öl-in-Wasser Emulsion*

Es wurden die in Tabelle 3 angegebenen Formulierungen mit Cellulosepartikeln aus Beispiel 1, ohne Cellulose und mit drei Typen mikrokristalliner Cellulose hergestellt und deren Viskosität, Stabilität, Erscheinungsbild und die Sensorik bei Verteilen auf der Haut bewertet.

Tabelle 4 gemäß Beispiel 4: Formulierungen und Ergebnisse von Beispiel 4 (mit Cellulosepartikeln aus Beispiel 1) und Vergleichsbeispielen V4.1 (ohne Cellulosepartikel), V4.2, V4.3 und V4.4 (jeweils mit Mikrokristalliner Cellulose), emulgatorfreie Öl-in-Wasser-Emulsion.

| Beispiel | 4 | V4.1 |
|---|---|---|
| TEGOSOFT® OP (Evonik Industries AG) (Ethylhexyl Palmitate) | 4,00 % | 4,00 % |
| TEGOSOFT® DEC (Evonik Industries AG) (Diethylhexylcarbonate) | 4,00 % | 4,00 % |
| TEGOSOFT® CT (Evonik Industries AG) (Caprylic/Capric Triglyceride) | 4,00 % | 4,00 % |
| TEGOSOFT® TN (Evonik Industries AG) (C12-15 Alkyl Benzoate) | 3,00 % | 3,00 % |
| TEGOSOFT® M (Evonik Industries AG) (Isopropyl Myristate) | 2,50 % | 2,50 % |
| Tocopheryl Acetate | 0,50 % | 0,50 % |
| TEGO® Carbomer 341 ER (Evonik Industries AG) (Acrylates/C10-30 Alkyl Acrylate Crosspolymer) | 0,30 % | 0,30 % |
| Cellulosepartikel aus Beispiel 1 | 1,00 % | 0,00 % |
| Glycerin | 3,00 % | 3,00 % |
| Wasser | ad 100 % | ad 100 % |
| Propylenglykol | 2,00 % | 2,00 % |
| Panthenol | 0,50 % | 0,50 % |

(fortgesetzt)

| Beispiel | 4 | V4.1 |
|---|---|---|
| 10%ige wässrige Natronlauge | 0,90 % | 0,90 % |
| Phenoxyethanol; Ethylhexylglycerin | q.a. | q.a. |
| | | |
| Viskosität Brookfield DV-I Prime Spindel 4 mit 5rpm | 28 Pas | 28 Pas |
| Stabilität | Gut | Gut |
| Erscheinungsbild | Weiß, homogen | Weiß, homogen |
| Sensorik beim Verteilen auf der Haut | Leicht zu verteilen, kaum ölig, gute Absorption. | Leicht zuverteilen, inhomogen, Wasser- und Ölphase getrennt auf der Haut. |

| Beispiel | V4.2 | V4.3 | V4.4 |
|---|---|---|---|
| TEGOSOFT® OP (Evonik Industries AG) (Ethylhexyl Palmitate) | 4,00 % | 4,00 % | 4,00 % |
| TEGOSOFT® DEC (Evonik Industries AG) (Diethylhexylcarbonate) | 4,00 % | 4,00 % | 4,00 % |
| TEGOSOFT® CT (Evonik Industries AG) (Caprylic/Capric Triglyceride) | 4,00 % | 4,00 % | 4,00 % |
| TEGOSOFT® TN (Evonik Industries AG) (C12-15 Alkyl Benzoate) | 3,00 % | 3,00 % | 3,00 % |
| TEGOSOFT® M (Evonik Industries AG) (Isopropyl Myristate) | 2,50 % | 2,50 % | 2,50 % |
| Tocopheryl Acetate | 0,50 % | 0,50 % | 0,50 % |
| TEGO® Carbomer 341 ER (Evonik Industries AG) (Acrylates/C10-30 Alkyl Acrylate Cross polymer) | 0,30 % | 0,30 % | 0,30 % |
| AVICEL CL-611 (FMC BioPolymer) (Microcristalline Cellulose) | 1,00 % | 0,00 % | 0,00 % |
| AVICEL PC-611 (FMC BioPolymer) (Microcristalline Cellulose, Cellulose Gum) | 0,00 % | 1,00 % | 0,00 % |
| AVICEL PH-101 (FMC BioPolymer) (Microcristalline Cellulose) | 0,00 % | 0,00 % | 1,00 % |
| Glycerin | 3,00 % | 3,00 % | 3,00 % |
| Wasser | ad 100 % | ad 100 % | ad 100 % |
| Propylenglykol | 2,00 % | 2,00 % | 2,00 % |
| Panthenol | 0,50 % | 0,50 % | 0,50 % |
| 10%ige wässrige Natronlauge | 0,90 % | 0,90 % | 0,90 % |
| Phenoxyethanol; Ethylhexylg lycerin | q.a. | q.a. | q.a. |
| Viskosität Brookfield DV-I Prime Spindel 4 mit 5rpm | 34 Pas | 37 Pas | >40 Pas (zu viskos, nicht messbar) |
| Viskosität Brookfield DV-I Prime Spindel c mit 10rpm | | | 20 Pas |

(fortgesetzt)

| Beispiel | V4.2 | V4.3 | V4.4 |
|---|---|---|---|
| Stabilität | Gut | Gut | Gut |
| Erscheinungsbild | Weiß, homogen | Weiß, homogen | Weiß, homogen |
| Sensorik beim Verteilen auf der Haut | Schlecht zu verteilen, Wasser- und Ölphase getrennt auf der Haut. | Schlecht zu verteilen, schmierig, inhomogen, Wasser- und Ölphase getrennt auf der Haut. | Schmierig, inhomogen, Wasser- und Ölphase getrennt auf der Haut. |

*Weitere Formulierungsbeispiele:*

Natürliche Wasser-in-Öl Creme

| | |
|---|---|
| ISOLAN® PDI (Evonik Industries AG) (Diisostearoyl Polyglyceryl-3 Dimer Dilinoleate) | 3,00 % |
| Castor Wachs | 0,40 % |
| Bienenwachs | 0,60 % |
| TEGOSOFT® CT (Evonik Industries AG) (Caprylic/Capric Triglyceride) | 7,00 % |
| TEGOSOFT® OER (Evonik Industries AG) (Oleyl Erucrate) | 4,00 % |
| TEGOSOFT® TIS (Evonik Industries AG) (Triisostearin) | 2,00 % |
| TEGOSOFT® CR (Evonik Industries AG) (Cetyl Ricinoleate) | 1,00 % |
| Avocadoöl | 5,00 % |
| Cellulosepartikel aus Beispiel 1 | 1,00 % |
| Glycerin | 7,00 % |
| Wasser | ad 100 % |
| Magnesiumsulfat Heptahydrat | 1,00 % |
| Euxyl K 712 (Schülke&Meyer) (Aqua, Sodium Benzoate, Potassium Sorbate) | 0,50 % |
| 10%ige wässrige Zitronensäure | pH-Wert auf 5 einstellen |

Öl-in-Wasser Lotion mit pH=4, nicht erfindungsgemäß

| | |
|---|---|
| ABIL® Care XL 80 (Evonik Industries AG) | 2,50 % |
| TEGOSOFT® Liquid (Evonik Industries AG) | 5,00 % |
| TEGOSOFT® DC (Evonik Industries AG) (Decyl Cocoate) | 5,00% |
| TEGOSOFT® M (Evonik Industries AG) (Isopropyl Myristate) | 5,00 % |
| Sepigel 305 (SEPPIC) (Polyacrylamide and C13-14 Isoparaffin and Laureth-7) | 1,50 % |
| Cellulosepartikel aus Beispiel 1 | 1,00 % |
| | |
| Wasser | ad 100 % |
| Propylen Glykol | 3,00 % |
| 10%ige wässrige Zitronensäure | Auf pH=4 einstellen |
| Methylisothiazolinone, Methylparaben Ethylparaben | q.a. |

Wasser-in-Öl Serum

| ABIL® EM 90 (Evonik Industries AG) (Cetyl PEG/PPG-10/1 Dimethicone) | 1,50 % |
|---|---|
| ABIL® EM 97 S (Evonik Industries AG) (Bis-PEG/PPG-14/14 Dimethicone, Dimethicone) | 1,00 % |
| Cyclopentasiloxane | 12,00 % |
| TEGOSOFT® DEC (Evonik Industries AG) (Diethylhexyl Carbonate) | 3,00 % |
| HyaCare® Filler CL (Evonik Industries AG) (Aqua; Ethylhexyl Stearate; Sodium Hyaluronate Crosspolymer; Polyglyceryl-4 Diisostearate/Polyhydroxystearate/ Sebacate; Sodium Isostearate) | 2,50 % |
| Tocopherol | 0,50 % |
| Zinc Stearate | 0,50 % |
| Wasser | ad 100 % |
| Glycerin | 4,00 % |
| Butylen Glykol | 4,00 % |
| Natriumchlorid | 0,80 % |
| TEGO® PEP 4-17 (Evonik Industries AG) (Tetrapeptide-21; Glycerin; Butylene Glycol; Aqua) | 0,50 % |
| Cellulosepartikel aus Beispiel 1 | 1,00 % |
| Phenoxyethanol; Ethylhexylglycerin | q.a. |

Öl-in-Wasser AP/Deo Lotion für Roll on Applikation, nicht erfindungsgemäß

| TEGO® Care PS (Evonik Industries AG) (Methyl Glucose Sesquistearate) | 1,75% |
|---|---|
| TEGO® Care PL 4 (Evonik Industries AG) (Polyglyceryl-4 Laurate) | 0,25% |
| TEGOSOFT® DEC (Evonik Industries AG) (Diethylhexyl Carbonate) | 3,00 % |
| TEGOSOFT® PBE (Evonik Industries AG) (PPG-14 Butyl Ether) | 3,00 % |
| TEGO® Cosmo P 813 (Evonik Industries AG) (Polyglyceryl-3-Caprylate) | 0,50 % |
| Cellulosepartikel aus Beispiel 1 | 1,00 % |
| Wasser | ad 100 % |
| Natrosol 250HHR (Hercules) (Hydroxyethylcellulose) | 1,00 % |
| 50%ige wässrige Aluminiumchlorohydrat Lösung | 15,00 % |
| Methylisothiazolinone, Methylparaben Ethylparaben | q.a. |

Öl-in-Wasser Men's Care Gel

| TEGOSOFT® AC (Evonik Industries AG) (Isoamyl Cocoate) | 2,00 % |
|---|---|
| TEGOSOFT® OER (Evonik Industries AG) (Oleyl Erucrate) | 2,00 % |
| TEGOSOFT® TIS (Evonik Industries AG) (Triisostearin) | 2,00 % |
| Cellulosepartikel aus Beispiel 1 | 1,00 % |
| Wasser | ad 100 % |
| Glycerin | 5,00 % |
| TEGO® Carbomer 341 ER (Evonik Industries AG) (Acrylates/C10-30 Alkyl Acrylate Crosspolymer) | 0,50 % |
| HyaCare® 50 (Evonik Industries AG) (Hydrolyzed Hyaluronic Acid) | 0,10 % |
| 10%ige wässrige Natronlauge | 1,50 % |
| Methylisothiazolinone, Methylparaben Ethylparaben | q.a. |

Tränklösung für Feuchttücher

| | |
|---|---|
| TEGO ®Wipe Flex (Evonik Industries AG) (Ethylhexylstearat, Phenoxyethanol, Polyglyceryl-4-laurat, Sorbitanlaurat, Dilaurylcitrat) | 5,70% |
| Cyclomethicone | 2,00% |
| Cellulosepartikel aus Bsp. 1 | 1,00% |
| Wasser | ad 100% |
| Glycerin | 3,00% |
| TEGO® Carbomer 141 (Evonik Industries AG) (Carbomer) | 0.10%. |
| Natriumhydroxid (10% in Wasser) | q.s. |

Make-Up Powder Foundation, nicht erfindungsgemäß

| | |
|---|---|
| Zinkstearat | 3,00% |
| Glimmer | ad 100 % |
| Talkum | 24,00% |
| Eisenoxid | 5,00% |
| Cellulosepartikel aus Beispiel 1 | 10,00% |
| Titandioxid | 8,00% |
| Cetylethylhexanoat | 2,00% |
| Squalan | 3,00% |
| Cetearylethylhexanoat | 2,00%. |
| Mineralöl (30 mPas) | 2,00% |
| PEG/PPG-4/12 Dimethicone | 1,00% |
| Aluminium Stärke Octernylsuccinat | q.s. |
| Eisenoxid | q.s. |
| Parfum | q.s. |

Make-Up Foundation, nicht erfindungsgemäß

| | |
|---|---|
| Phenyltrimethicone | 14,00% |
| Ethylhexylpalmitat | ad 100 % |
| Cetylethylhexanoat | 5,00% |
| Carnaubawachs | 4,70% |
| Stearoxydimethicone | 4,00% |
| PVP/Eicosene Copolymer | 1,00% |
| Cetylstearylheptanoat | 2,85% |
| Covabead LH 85, Polymethylmethacrylat Partikel | 3,00% |
| Siliciumdioxid | 0,25%. |
| Zinkoxid | 7,00% |
| Nylon-1010 | 2,50% |
| Talc Covasil 4.05 | 9,50% |

(fortgesetzt)

| | |
|---|---|
| Acrylat Copolymer | 2,00% |
| Cellulosepartikel aus Beispiel 1 | 2,50% |
| Aluminium Stärke Octernylsuccinat | 9,50% |
| Eisenoxid | 3,10% |
| Titandioxid (und) Dimethicone | 14,50% |

Lidschattenformulierung, nicht erfindungsgemäß

| | |
|---|---|
| Cyclopentasiloxane | ad 100% |
| PPG-3 Myristylether | 7,00% |
| ABIL® WE 09 (Evonik Industries AG) (Polyglyceryl-4-Isostearat; Cetyl PEG/PPG-10/1 Dimethicone; Hexyllaurat) | 1,00% |
| Dimethicone (20 mPas) | 2,50% |
| Cera Alba | 4,50% |
| Carnaubawachs | 2,00% |
| A-C Copolymer 400 (Ethylen/VA Copolymer) | 2,50% |
| Ozokerit | 5,80% |
| C18-36-Säuretriglycerid | 2,00% |
| Liquiparöl (Isobutylparaben (und) Isopropylparaben (und) Butylparaben) | 0,20% |
| Cellulosepartikel aus Beispiel 1 | 4,00% |
| Titandioxid | 5,00% |
| Chromoxid Grün) | 10,00% |
| CI 77491 (und) Aluminium Puder (und) Siliciumdioxid | 5,00% |
| CI 77891 (und) CI 77288 (und) Glimmer | 10,00% |

Feuchtigkeitsspendende Öl-in-Wasser Sonnenschutzlotion mit hohem Sonnenschutzfaktor

| | |
|---|---|
| TEGO® Care PS (Evonik Industries AG) (Methyl Glucose Sesquistearate) | 2,50 % |
| TEGO® Alkanol 1618 (Evonik Industries AG) (Cetearylalkohol) | 0,75 % |
| TEGOSOFT® XC (Evonik Industries AG) (Phenoyethylcaprylat) | 4,85 % |
| TEGOSOFT® DEC (Evonik Industries AG) (Diethylhexylcarbonat) | 2,00 % |
| Butyloctyl Salicilate | 2,00 % |
| Octocrylene | 2,00 % |
| Butyl Methoxydibenzoylmethane | 8,00 % |
| Bis-Ethylhexyloxyphenol Methoxyphenyl Triazine | 2,00 % |
| Ethylhexyl Salicylate | 3,00 % |
| Ethylhexyl Triazone | 2,00 % |
| Glycerin | 4,00 % |
| LACTIL® (Evonik Industries AG) (Sodium Lactate; Sodium PCA; Glycine; Fructose; Urea; Niacinamide; Inositol; Sodium Benzoate; Lactic Acid) | 2,00 % |
| Wasser | ad 100 % |

(fortgesetzt)

| Cellulosepartikel aus Beispiel 1 | 1,00 % |
|---|---|
| TEGO® Carbomer 341 ER (Evonik Industries AG) (Acrylates/C10-30 Alkyl Acrylate Crosspolymer) | 0,15 % |
| Natriumhydroxid (10%-ig in Wasser) | 0,40 % |
| Methylisothiazolinone, Methylparaben; Ethylparaben | q.a. |
| Parfum | q.a. |

Lippenstiftformulierung, nicht erfindungsgemäß

| Cyclopentasiloxan | 34,00% |
|---|---|
| Behenoxydimethicone | 3,00% |
| Stearyldimethicone | 10,00% |
| Polyisobuten | 5,00% |
| Phenyltrimethicone | 8,00% |
| Isododecan | 4,00% |
| Bis-Diglyceryl Polyacyladipat-2 | 4,00% |
| Ceresin | 24,00% |
| Titandioxid | 1,00% |
| Karminrot | 1,00% |
| D&C Red Nr. 7 | 3,00% |
| Polyethylen | 1,00% |
| Cellulosepartikel aus Beispiel 1 | 1,00% |
| Aluminium Stärke Octenylsuccinat & Lauroyllysin | 1,00% |

Mascaraformulierung, nicht erfindungsgemäß

| Sucrosestearat | 4,00% |
|---|---|
| Polyglyceryl-3 Methylglucosedistearat | 2,00% |
| Stearylalkohol | 1,00% |
| Cadelillawachs | 5,00% |
| Carnaubawachs | 1,75% |
| Bienenwachs | 4,25% |
| Hydriertes Reiskleiewachs | 5,00% |
| Adipinsäure/Diethylenglycol/Glycerin Crosspolymer | 5,00% |
| Ceramid NP | 0,05% |
| Eisenoxid | 10,00% |
| Cellulosepartikel aus Beispiel 1 | 0,50% |
| Wasser | ad 100% |
| 1,3-Butandiol | 3,00% |
| Triethanolamin | 1,80% |
| Acrylate/Octylacrylamid Copolymer | 5,00% |

(fortgesetzt)

| Phenoxyethanol; Methylparaben; Ethylparaben, Butylparaben; Propylparaben, Isobutylparaben | 0,60% |
|---|---|

**Patentansprüche**

1. Kosmetische Formulierung enthaltend
Glycerin und feste Partikel mit einer mittleren Partikelgröße von 3 $\mu$m bis 15 $\mu$m, besonders bevorzugt von 4 $\mu$m bis 10 $\mu$m,
**dadurch gekennzeichnet,**
**dass** die Partikel mindestens 95 Gew.-%, bevorzugt mindestens 97 Gew.-%, besonders bevorzugt mindestens 99 Gew.-% native, aus Pflanzenfasern gewonnene Cellulose enthalten, wobei sich die Gewichtsprozente auf das trockene Partikelgesamtgewicht beziehen, und
**dass** sie eine Emulsion ist.

2. Kosmetische Formulierung gemäß Anspruch 1, **dadurch gekennzeichnet, dass** sie bezogen auf die Gesamtformulierung von 0,001 Gew.-% bis 30 Gew.-%, bevorzugt 0,01 Gew.-% bis 20 Gew.-%, besonders bevorzugt 0,1 Gew.-% bis 10 Gew.-% der Partikel enthält.

3. Kosmetische Formulierung gemäß Anspruch 1 oder 2, **dadurch gekennzeichnet, dass** die Partikel bevorzugt eine Cellulose eines Kristallinitätsgrades von 40 bis 90 % aufweisen.

4. Kosmetische Formulierung gemäß mindestens einem der vorherigen Ansprüche , **dadurch gekennzeichnet, dass** die Partikel eine maximale Löslichkeit in Wasser bei pH 7,0, 20 °C, 1 bar, von 0 g/l bis 0,5 g/l, bevorzugt von 0 g/l bis 0,2 g/l, besonders bevorzugt von 0 g/l bis 0,08 g/l aufweisen.

5. Kosmetische Formulierung gemäß mindestens einem der vorherigen Ansprüche, **dadurch gekennzeichnet, dass** die in den Partikeln enthaltene Cellulose einen mittleren Polymerisierungsgrad von 1 bis 50000.

6. Kosmetische Formulierung gemäß mindestens einem der vorherigen Ansprüche, **dadurch gekennzeichnet, dass** die enthaltenen festen Partikel eine Schüttdichte von 100-300 g/L, bevorzugt 120-270 g/L, besonders bevorzugt 140-240 g/L besitzen

7. Kosmetische Formulierung gemäß mindestens einem der vorherigen Ansprüche, **dadurch gekennzeichnet, dass** sie eine O/W- oder eine W/O-Emulsion mit einer Viskosität bei einer Scherrate von 10 s$^{-1}$ und bei 20 °C von 0,01 Pas bis 100000 Pas, bevorzugt von 1 Pas bis 20000 Pas, besonders bevorzugt von 10 Pas, insbesondere 25 Pas, bis 10000 Pas ist.

8. Kosmetische Formulierung gemäß mindestens einem der vorherigen Ansprüche, **dadurch gekennzeichnet, dass** die Partikel ein Ölabsorptionsvermögen von von 1,0 g bis 2,5 g, bevorzugt von 1,2 g bis 2,2 g, besonders bevorzugt von 1,5 g bis 2,0 g Cyclopentasiloxane pro g trockenem Partikel aufweisen.

9. Kosmetische Formulierung gemäß mindestens einem der vorherigen Ansprüche, **dadurch gekennzeichnet, dass** die Partikel ein Ölabsorptionsvermögen von 1,0 g bis 2,0 g, bevorzugt von 1,2 g bis 1,8 g, besonders bevorzugt von 1,5 g bis 1,7 g Diethyhexylcarbonate pro g trockenem Partikel aufweisen.

10. Kosmetische Formulierung gemäß mindestens einem der vorherigen Ansprüche, **dadurch gekennzeichnet, dass** die Partikel ein Ölabsorptionsvermögen von 1,0 g bis 2,0 g, bevorzugt von 1,1 g bis 1,7 g, besonders bevorzugt von 1,4 g bis 1,6 g Isopropyl-Myristate pro g trockenem Partikel aufweisen.

11. Kosmetische Formulierung gemäß mindestens einem der vorherigen Ansprüche, **dadurch gekennzeichnet, dass** die Partikel ein Ölabsorptionsvermögen von 1,0 g bis 2,0 g, bevorzugt von 1,2 g bis 1,8 g, besonders bevorzugt von 1,45 g bis 1,7 g Caprylic/Capric Triglyceride pro g trockenem Partikel aufweisen.

12. Kosmetische Formulierung gemäß mindestens einem der vorherigen Ansprüche, **dadurch gekennzeichnet, dass** die Partikel ein Ölabsorptionsvermögen von 1,0 g bis 2,5 g, bevorzugt von 1,2 g bis 2,2 g, besonders bevorzugt von 1,5 g bis 2,0 g Mineralöl pro g trockenem Partikel aufweisen.

13. Kosmetische Formulierung gemäß mindestens einem der vorherigen Ansprüche, **dadurch gekennzeichnet, dass** die Partikel ein Wasserabsorptionsvermögen von 1 g bis 3 g, besonders bevorzugt von 1,7 g bis 2,3 g Wasser von pH 7 pro g trockenem Partikel aufweisen.

14. Verwendung von festen Partikel mit einer mittleren Partikelgröße von 3 μm bis 15 μm, besonders bevorzugt von 4 μm bis 10 μm, zur Herstellung einer kosmetischen Formulierung nach mindestens einem der vorherigen Ansprüche, **dadurch gekennzeichnet, dass** die Partikel zu mindestens 95 Gew.-%, bevorzugt mindestens 97 Gew.-%, besonders bevorzugt mindestens 99 Gew.-% aus nativer, aus Pflanzenfasern gewonnener Cellulose bestehen, wobei sich die Gewichtsprozent auf das trockene Partikelgesamtgewicht beziehen.

**Claims**

1. Cosmetic formulation containing
   glycerol and
   solid particles having a mean particle size of 3 μm to 15 μm, particularly preferably of 4 μm to 10 μm, **characterized in that** the particles contain at least 95% by wt., preferably at least 97% by wt., particularly preferably at least 99% by wt., native cellulose obtained from plant fibres, the percentages by weight relating to the dry total particle weight, and that the formulation is an emulsion.

2. Cosmetic formulation according to Claim 1, **characterized in that** it contains, based on the total formulation, from 0.001% by wt. to 30% by wt., preferably 0.01% by wt. to 20% by wt., particularly preferably 0.1% by wt. to 10% by wt., of the particles.

3. Cosmetic formulation according to Claim 1 or 2, **characterized in that** the particles preferably contain a cellulose of a degree of crystallinity of 40 to 90%.

4. Cosmetic formulation according to at least one of the preceding claims, **characterized in that** the particles have a maximal solubility in water at pH 7.0, 20°C, 1 bar, of 0 g/l to 0.5 g/l, preferably of 0 g/l to 0.2 g/l, particularly preferably of 0 g/l to 0.08 g/l.

5. Cosmetic formulation according to at least one of the preceding claims, **characterized in that** the cellulose contained in the particles a mean degree of polymerization of 1 to 50000.

6. Cosmetic formulation according to at least one of the preceding claims, **characterized in that** the solid particles contained have a bulk density of 100-300 g/L, preferably 120-270 g/L, particularly preferably 140-240 g/L.

7. Cosmetic formulation according to at least one of the preceding claims, **characterized in that** it is an O/W oder a W/O emulsion, having a viscosity at a shear rate of 10 s$^{-1}$ and at 20°C of 0.01 Pas to 100000 Pas, preferably of 1 Pas to 20000 Pas, particularly preferably of 10 Pas, in particular 25 Pas, to 10000 Pas.

8. Cosmetic formulation according to at least one of the preceding claims, **characterized in that** the particles have an oil absorption power of of 1.0 g to 2.5 g, preferably of 1.2 g to 2.2 g, particularly preferably of 1.5 g to 2.0 g of cyclopentasiloxane per g of dry particles.

9. Cosmetic formulation according to at least one of the preceding claims, **characterized in that** the particles have an oil absorption power of 1.0 g to 2.0 g, preferably of 1.2 g to 1.8 g, particularly preferably of 1.5 g to 1.7 g, of diethyhexyl carbonate per g of dry particles.

10. Cosmetic formulation according to at least one of the preceding claims, **characterized in that** the particles have an oil absorption power of 1.0 g to 2.0 g, preferably of 1.1 g to 1.7 g, particularly preferably of 1.4 g to 1.6 g of isopropyl myristate per g of dry particles.

11. Cosmetic formulation according to at least one of the preceding claims, **characterized in that** the particles have an oil absorption power of 1.0 g to 2.0 g, preferably of 1.2 g to 1.8 g, particularly preferably of 1.45 g to 1.7 g of caprylic/capric triglycerides per g of dry particles.

**12.** Cosmetic formulation according to at least one of the preceding claims, **characterized in that** the particles have an oil absorption power of 1.0 g to 2.5 g, preferably of 1.2 g to 2.2 g, particularly preferably of 1.5 g to 2.0 g, of mineral oil per g of dry particles.

**13.** Cosmetic formulation according to at least one of the preceding claims, **characterized in that** the particles have a water absorption power of 1 g to 3 g, preferably of 1.5 g to 3.5 g, particularly preferably of 1.7 g to 2.3 g, of water of pH 7 per g of dry particles.

**14.** Use of solid particles having an average particle size of 3 $\mu$m to 15 $\mu$m, particularly preferably of 4 $\mu$m to 10 $\mu$m, for the production of a cosmetic formulation according to at least one of the preceding claims, **characterized in that** the particles consist to at least 95% by wt., preferably at least 97% by wt., particularly preferably at least 99% by wt., of native cellulose obtained from plant fibres, the percentages by weight relating to the dry total particle weight.

**Revendications**

**1.** Formulation cosmétique, contenant :

de la glycérine et
des particules solides ayant une taille de particule moyenne de 3 $\mu$m à 15 $\mu$m, de manière particulièrement préférée de 4 $\mu$m à 10 $\mu$m,
**caractérisée en ce que**
les particules contiennent au moins 95 % en poids, de préférence au moins 97 % en poids, de manière particulièrement préférée au moins 99 % en poids, de cellulose naturelle, obtenue à partir de fibres végétales, les pourcentages en poids se rapportant au poids total des particules sèches, et
**en ce qu'**il s'agit d'une émulsion.

**2.** Formulation cosmétique selon la revendication 1, **caractérisée en ce qu'**elle contient, par rapport à la formulation totale, de 0,001 % en poids à 30 % en poids, de préférence 0,01 % en poids à 20 % en poids, de manière particulièrement préférée 0,1 % en poids à 10 % en poids, des particules.

**3.** Formulation cosmétique selon la revendication 1 ou 2, **caractérisée en ce que** les particules comprennent de préférence une cellulose ayant un degré de cristallinité de 40 à 90 %.

**4.** Formulation cosmétique selon au moins l'une quelconque des revendications précédentes, **caractérisée en ce que** les particules présentent une solubilité maximale dans l'eau à pH 7,0, 20 °C, 1 bar, de 0 g/l à 0,5 g/l, de préférence de 0 g/l à 0,2 g/l, de manière particulièrement préférée de 0 g/l à 0,08 g/l.

**5.** Formulation cosmétique selon au moins l'une quelconque des revendications précédentes, **caractérisée en ce que** la cellulose contenue dans les particules un degré de polymérisation moyen de 1 à 50 000.

**6.** Formulation cosmétique selon au moins l'une quelconque des revendications précédentes, **caractérisée en ce que** les particules solides contenues présentent une densité apparente de 100 à 300 g/l, de préférence de 120 à 270 g/l, de manière particulièrement préférée de 140 à 240 g/l.

**7.** Formulation cosmétique selon au moins l'une quelconque des revendications précédentes, **caractérisée en ce qu'**il s'agit d'une émulsion H/E ou E/H ayant une viscosité à un taux de cisaillement de 10 s$^{-1}$ et à 20 °C de 0,01 Pas à 100 000 Pas, de préférence de 1 Pas à 20 000 Pas, de manière particulièrement préférée de 10 Pas, notamment de 25 Pas, à 10 000 Pas.

**8.** Formulation cosmétique selon au moins l'une quelconque des revendications précédentes, **caractérisée en ce que** les particules présentent une capacité d'absorption de de l'huile de 1,0 g à 2,5 g, de préférence de 1,2 g à 2,2 g, de manière particulièrement préférée de 1,5 g à 2,0 g de cyclopentasiloxanes par g de particules sèches.

**9.** Formulation cosmétique selon au moins l'une quelconque des revendications précédentes, **caractérisée en ce que** les particules présentent une capacité d'absorption de l'huile de 1,0 g à 2,0 g, de préférence de 1,2 g à 1,8 g, de manière particulièrement préférée de 1,5 g à 1,7 g de carbonate de diéthylhexyle par g de particules sèches.

**10.** Formulation cosmétique selon au moins l'une quelconque des revendications précédentes, **caractérisée en ce que** les particules présentent une capacité d'absorption de l'huile de 1,0 g à 2,0 g, de préférence de 1,1 g à 1,7 g, de manière particulièrement préférée de 1,4 g à 1,6 g de myristate d'isopropyle par g de particules sèches.

**11.** Formulation cosmétique selon au moins l'une quelconque des revendications précédentes, **caractérisée en ce que** les particules présentent une capacité d'absorption de l'huile de 1,0 g à 2,0 g, de préférence de 1,2 g à 1,8 g, de manière particulièrement préférée de 1,45 g à 1,7 g de triglycéride caprylique/caprique par g de particules sèches.

**12.** Formulation cosmétique selon au moins l'une quelconque des revendications précédentes, **caractérisée en ce que** les particules présentent une capacité d'absorption de l'huile de 1,0 g à 2,5 g, de préférence de 1,2 g à 2,2 g, de manière particulièrement préférée de 1,5 g à 2,0 g d'huile minérale par g de particules sèches.

**13.** Formulation cosmétique selon au moins l'une quelconque des revendications précédentes, **caractérisée en ce que** les particules présentent une capacité d'absorption de l'eau de 1 g à 3 g, de manière particulièrement préférée de 1,7 g à 2,3 g d'eau de pH 7 par g de particules sèches.

**14.** Utilisation de particules solides ayant une taille de particule moyenne de 3 $\mu$m à 15 $\mu$m, de manière particulièrement préférée de 4 $\mu$m à 10 $\mu$m, pour la fabrication d'une formulation cosmétique selon au moins l'une quelconque des revendications précédentes, **caractérisée en ce que** les particules sont constituées par au moins 95 % en poids, de préférence au moins 97 % en poids, de manière particulièrement préférée au moins 99 % en poids, de cellulose naturelle, obtenue à partir de fibres végétales, les pourcentages en poids se rapportant au poids total des particules sèches.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1036799 A
- EP 0264853 A
- EP 1545433 A
- WO 2009085444 A
- EP 1299069 A
- EP 1372576 A
- WO 2009112375 A
- DE 102008001788

**In der Beschreibung aufgeführte Nicht-Patentliteratur**

- **N. TERINTE ; R. IBBETT ; K. C. SCHUSTER.** *Lenzinger Berichte,* 2011, vol. 89, 118-131
- **PARK et al.** *Biotechnology for Biofuels,* 2010, vol. 3, 10